# EUROPÄISCHE PATENTANMELDUNG

(11) **EP 4 649 911 A2**
(43) Veröffentlichungstag der Anmeldung: **19.11.2025**
(21) Anmeldenummer: 25171878.9
(22) Anmeldetag: 23.04.2025
(51) Int. Cl.: A61B 34/20

(54) **STATIONÄRE MEDIZINISCHE RESEKTIONSVORRICHTUNG ZUR MASCHINENGEFÜHRTEN BESTIMMUNG EINES OBERFLÄCHENVERLAUFS EINER OBERFLÄCHE VON ZU RESEZIERENDEM GEWEBE**

(30) Priorität: 26.04.2024 LU 507049
(71) Anmelder: Universität des Saarlandes, 66123 Saarbrücken (DE)
(72) Erfinder: WINTER, Philipp, 66773 Schwalbach (DE); LANDGRAEBER, Stefan, 66424 Homburg (DE)
(74) Vertreter: Zeiner, Johannes Michael

(57) **Zusammenfassung**

Die Erfindung betrifft ein Verfahren zur maschinengeführten Bestimmung eines Oberflächenverlaufs einer Oberfläche (1) von zu resezierendem Gewebe (2), insbesondere eines Oberflächenverlaufs von zu resezierendem Knochengewebe, bei dem eine räumliche Position einzelner Messpunkte (5) der Oberfläche (1) des zu resezierenden Gewebes (2) bestimmt wird und aus der räumlichen Position der einzelnen Messpunkte der Oberflächenverlauf ermittelt wird. Zweckmäßigerweise wird der Oberflächenverlauf von einer stationären medizinischen Resektionsvorrichtung (3) ermittelt, die zur Resektion des zu resezierenden Gewebes (2) eingerichtet ist, oder der Oberflächenverlauf wird von einer stationären medizinischen Resektionsvorrichtung (3) ermittelt, die zur Bestimmung und Anzeige von Positionen (13) eingerichtet ist, an denen ein Resektionshilfsmittel zur teilweise manuellen Durchführung der Resektion anzubringen ist.

Ferner betrifft die Erfindung eine medizinische Resektionsvorrichtung (3), insbesondere einen Operationsroboter, zur Durchführung des erfindungsgemäßen Verfahrens.

## Beschreibung

Die Erfindung betrifft ein Verfahren zur maschinengeführten Bestimmung eines Oberflächenverlaufs einer Oberfläche von zu resezierendem Gewebe, insbesondere eines Oberflächenverlaufs von zu resezierendem Knochengewebe, bei dem eine räumliche Position einzelner Messpunkte der Oberfläche des zu resezierenden Gewebes bestimmt wird und aus der räumlichen Position der einzelnen Messpunkte der Oberflächenverlauf ermittelt wird.

Ferner betrifft die Erfindung eine stationäre medizinische Resektionsvorrichtung, insbesondere einen Operationsroboter, zur Durchführung des vorgenannten Verfahrens oder zur Durchführung eines Verfahrens zur Resektion von Gewebe, bei dem ein Oberflächenverlauf einer Oberfläche von zu resezierendem Gewebe von der medizinischen Resektionsvorrichtung bestimmt wird, indem eine räumliche Position einzelner Messpunkte der Oberfläche des zu resezierenden Gewebes bestimmt wird und aus der räumlichen Position der einzelnen Messpunkte der Oberflächenverlauf ermittelt wird, wobei wobei der von der medizinischen Resektionsvorrichtung ermittelte Oberflächenverlauf zu deren Ansteuerung zur Resektion des zu resezierenden Gewebes verwendet wird, oder wobei der von der medizinischen Resektionsvorrichtung ermittelte Oberflächenverlauf zur Bestimmung und Anzeige von Positionen eingerichtet ist, an denen ein Resektionshilfsmittel zur teilweise manuellen Durchführung der Resektion anzubringen ist.

Eine stationäre medizinische Resektionsvorrichtung im Sinn dieser Erfindung ist eine solche, die während der Bestimmung des Oberflächenverlaufs ortsfest angeordnet ist.

Eine stationäre medizinische Resektionsvorrichtung im Sinn dieser Erfindung ist außerdem nicht handgeführt, sondern folgt selbständig einem programmierten Ablauf zur Bestimmung des Oberflächenverlaufs.

Maschinegeführt bedeutet, dass die Bestimmung eines Oberflächenverlaufs einem programmierten Ablauf von der Resektionsvorrichtung ohne menschlichen Eingriff, jedoch unter menschlicher Überwachung, durchgeführt wird.

Eine maschinengeführte Bestimmung eines Oberflächenverlaufs einer Oberfläche von zu resezierendem Gewebe erfolgt durch die stationäre medizinische Resektionsvorrichtung. Dies bedeutet, dass zur Bestimmung des Oberflächenverlaufs keine manuelle Führung eines Messkopfs erfolgt, sondern ausschließlich eine maschinelle Führung. Im Fall eines Operationsroboters bedeutet dies, dass beispielsweise ein taktiler Messkopf an dem Operationsroboter befestigt ist, vorzugsweise lösbar, und ein Antasten der Oberfläche, deren Oberflächenverlauf zu bestimmen ist, durch eine Bewegung des Operationsroboters erfolgt und eben nicht durch eine manuelle, also handgeführte Bewegung des Messkopfes.

Ein Resektionshilfsmittel ist ein Mittel, durch das sichergestellt wird, dass eine Resektion genau an derjenigen Stelle erfolgt, an der diese von einem Operateur geplant ist. Eine solche Stelle kann beispielsweise eine Schnittebene sein, in der durch eine handgeführte Säge eine Resektion erfolgen soll.

Ein Resektionshilfmittel kann insbesondere ein sogenannter Schnittblock sein, der an einem Knochen, der teilweise reseziert werden soll, durch Knochenpins, die Befestigungspins sind, befestigt wird.

Ein Verfahren und eine handgeführte Vorrichtung zu Bestimmung eines Oberflächenverlaufs von zu resezierendem Gewebe und zu dessen Resektion sind aus US 2017/258532 A1 bekannt.

Zu resezierendes Gewebe kann jede Art von menschlichem Gewebe sein. Vorzugsweise ist das zu resezierende Gewebe Knochengewebe.

Bei Operationen, bei denen einer Person beispielsweise ein künstliches Kniegelenk eingesetzt wird, wird Knochengewebe derart reseziert, dass sich ein Implantat nahezu nahtlos in das Skelett der Person einfügt. Operateure werden dazu von Operationsrobotern unterstützt, die erforderliche Operationswerkzeuge präzise führen.

Dazu ist bisher ein zweistufiges Verfahren erforderlich, bei dem in einem ersten Verfahrensschritt mittels einer Referenzierungsvorrichtung zuerst eine Referenzierung des zu resezierenden Knochens erfolgt, das heißt eine Bestimmung von Position des Knochens im Raum sowie dessen Ausrichtung. Mittels des Operationsroboters erfolgt in einem zweiten Verfahrensschritt die eigentliche Resektion des Knochens zur Einbringung des Implantats.

Damit ein Operationsroboter eine Resektion an korrekten Stellen eines Knochens durchführen kann, sind aus dem Stand der Technik mehrere Verfahren zur Bestimmung einer räumlichen Position und Ausrichtung eines zu resezierenden Knochens bekannt.

Danach werden bei einem ersten bekannten Verfahren zwei bis vier sogenannte Knochenpins, die Infrarotreflektoren sind, in den freigelegten Knochen an markanten Knochenpunkten ortsfest eingebracht. Anhand weiterer, während einer Operation auf den Knochen aufgebrachter, sogenannter mobiler Infrarotreflektoren kann anhand eines Reflexionsbildes der Knochenpins und der mobilen Infrarotreflektoren ein Oberflächenverlauf des zu resezierenden Knochens ermittelt werden. Dieser Oberflächenverlauf kann in ein Bezugssystem eines Operationsroboters transformiert werden, so dass dieser an für die Resektion erforderlichen Stelle Operationswerkzeuge präzise einsetzen kann. Nachteilig bei diesem Verfahren ist, dass die Knochenpins, die üblicherweise einen Durchmesser von 3,2 mm aufweisen, zu einer erheblichen Beschädigung des Knochens, in den sie eingebracht werden, führen können.

Bei einem zweiten bekannten Verfahren wird ein Oberflächenverlauf anhand von bekannten computertomographischen Referenzbildern von zu dem zu resezierenden Knochen vergleichbaren Knochen und tomographischen Bildern des zu resezierenden Knochens geschätzt und in ein Bezugssystem des Operationsroboters transformiert.

Nachteilig ist, dass zwei getrennte Vorrichtungen zur Durchführung der Operation erforderlich sind, eine Vorrichtung zur Referenzierung und eine Vorrichtung, der eigentliche Operationsroboter, zur Führung der Operationswerkzeuge. Dadurch wird eine Operationszeit erheblich verlängert.

Der Erfindung liegt daher die Aufgabe zugrunde, ein Verfahren zur Bestimmung eines Oberflächenverlaufs einer Oberfläche von zu resezierendem Gewebe der eingangs genannten Art zu schaffen, das präzise und schnell durchführbar ist.

Ferner liegt der Erfindung die Aufgabe zugrunde, ein Verfahren zu schaffen, bei dem eine besonders genaue räumliche Position und räumliche Anordnung von zu resezierendem Gewebe ohne operativen Eingriff in das zu resezierende Gewebe möglich ist.

Erfindungsgemäß wird die Aufgabe dadurch gelöst, dass der Oberflächenverlauf von einer stationären medizinischen Resektionsvorrichtung ermittelt wird, die zur Resektion des zu resezierenden Gewebes eingerichtet ist, oder dass der Oberflächenverlauf von einer stationären medizinischen Resektionsvorrichtung ermittelt wird, die zur Bestimmung und Anzeige von Positionen eingerichtet ist, an denen ein Resektionshilfsmittel zur teilweisen manuellen Durchführung der Resektion anzubringen ist.

Eine stationäre medizinische Resektionsvorrichtung ist vorzugsweise ein Operationsroboter, der zur Durchführung von Operationen an Menschen oder Tieren zugelassen ist. Ein Operationsroboter kann beispielsweise ein zur Verwendung für chirurgische Eingriffe zugelassener Sechsachsroboter sein.

Dadurch, dass der Oberflächenverlauf von der stationären medizinischen Resektionsvorrichtung bestimmt wird, ist vorteilhaft nur eine einzige Vorrichtung zur präzisen Durchführung einer Geweberesektion erforderlich, da Daten des Oberflächenverlaufs unmittelbar von der medizinischen Resektionsvorrichtung ermittelt, ausgewertet und verwendet werden können.

Ferner liegt der Erfindung die Aufgabe zugrunde, eine Vorrichtung auszubilden, durch die ein einstufiges Resektionsverfahren schaffen geschaffen wird, bei dem beide Verfahrensschritte, das heißt eine Bestimmung einer Position und Ausrichtung des zu resezierenden Gewebes und die eigentliche Resektion, mittels einer einzigen, das heißt ein und derselben Vorrichtung durchführbar sind, oder zumindest ein Bereich von resezierendem Gewebe, in dem ein ein Operateur eine Resektionshilfsmittel zur Durchführung eines manuellen Schnitts anbringen muss, durch die Vorrichtung bestimmbar und anzeigbar ist.

Erfindungsgemäß wird diese Aufgabe durch eine medizinische Resektionsvorrichtung nach Anspruch 11 gelöst.

Die Erfinder haben erkannt, dass eine Referenzierung des Knochens durch die medizinische Resektionsvorrichtung als solche möglich ist, wodurch ein für einen Patienten besonders schonendes Operationsverfahren geschaffen wird, bei dem keine Knochenpins zur Referenzierung in einen Knochen einoperiert werden müssen.

Zweckmäßigerweise wird aus dem von der stationären medizinischen Resektionsvorrichtung ermittelten Oberflächenverlauf eine dreidimensionale Ansicht des zu resezierenden Gewebes erstellt.

Die dreidimensionale Ansicht kann von einem Operateur zur Programmierung der medizinischen Resektionsvorrichtung und zur Überwachung von deren Arbeit genutzt werden. Dadurch, dass die stationäre medizinische Resektionsvorrichtung sowohl zur Ermittlung des Oberflächenverlaufs als auch zur Resektion oder zur Bestimmung und Anzeige von Positionen eingerichtet ist, an denen ein Resektionshilfsmittel zur teilweisen manuellen Durchführung der Resektion anzubringen ist, einsetzbar ist, ist keine Transformation von Raumkoordinaten in Bezugssysteme unterschiedlicher Vorrichtungen erforderlich. Fehler, die durch Koordinatentransformation oder numerische Rundungsfehler entstehen, werden vorteilhaft verhindert.

In einer Ausgestaltung der Erfindung wird die räumliche Position der einzelnen Messpunkte der Oberfläche taktil bestimmt.

Vorteilhaft ist eine besonders einfache Bestimmung der Messpunkte möglich. Dazu kann die stationäre medizinische Resektionsvorrichtung statt mit Operationswerkzeug mit einem taktilen Messkopf bestückt werden. Deren Doppelverwendung wird damit möglich, insbesondere wenn die medizinische Resektionsvorrichtung ein Operationsroboter ist.

Durch die taktile Bestimmung wird ein besonders schonendes Verfahren ausgebildet, durch das eine Beschädigung von zu resezierendem Gewebe verhindert wird.

In einer Ausgestaltung der Erfindung wird die räumliche Position der einzelnen Messpunkte und/oder der Oberflächenverlauf in einem Bezugssystem der stationären medizinischen Resektionsvorrichtung bestimmt.

Vorteilhaft können mit einer einzigen medizinischen Resektionsvorrichtung eine Referenzierung und ein Eingriff, das heißt eine Resektion von zu resezierendem Gewebe, durchgeführt werden.

Weiter vorteilhaft ist keine Koordinatentransformation aus einem Bezugssystem einer Referenziervorrichtung in ein Bezugssystem der medizinischen Resektionsvorrichtung erforderlich.

In einer Ausgestaltung der Erfindung werden benachbarte Messpunkte in einem Bezugssystem der medizinischen Resektionsvorrichtung derart ausgewählt, dass diese einen Abstand zwischen 1,00 mm und 6,00 mm aufweisen, vorzugsweise zwischen 2,00 mm und 5,00 mm. Die Erfinder haben festgestellt, dass ein Abstand zwischen 1,00 und 6,00 mm einen genauen, das heißt realitätsnahen Oberflächenverlauf ermöglicht. Eine besonders hohe Genauigkeit wurde für einen Abstand von 2,00 mm bis 5,00 mm gefunden, wobei dies überaschenderweise gewebeunabhängig war.

Ein Abstand von weniger als 1,00 mm erhöht eine Zeit zur Bestimmung des Oberflächenverlaufs erheblich ohne signifikanten Zugewinn bei der Genauigkeit, während bei einem Abstand von mehr als 6,00 mm überraschenderweise ein Genauigkeitsabfall beobachtet wurde, der dazu führt, dass das Verfahren für medizinische Zwecke mangels Präzision ungeeignet ist.

In einer weiteren Ausgestaltung der Erfindung wird eine Referenzebene bestimmt, die von dem zu resezierenden Gewebe beabstandet ist, und von der aus die räumliche Position der einzelnen Messpunkte der Oberfläche des zu resezierenden Gewebes bestimmt wird.

Zur Bildung der Referenzebene werden mindestens drei Punkte auf einer ebenen Platte taktil angefahren. Anhand dieser mindestens drei Punkte wird die Referenzebene gebildet.

Die ebene Platte ist im Raum in unmittelbarer Nähe zu dem zu resezierenden Gewebe angeordnet und Koordinaten von deren räumlicher Anordnung sind im Bezugssystem der stationären medizinischen Resektionsvorrichtung hinterlegt, das heißt bekannt.

Ein gewählter Punkt auf der ebenen Platte kann beispielsweise als sogenannter Nullpunkt herangezogen werden.

Dadurch, dass sich die Referenzebene in unmittelbarer Nähe zu dem zu resezierenden Gewebe befindet, kann diese, insbesondere der gewählte Nullpunkt, als Ausgangspunkt verwendet werden, um ein Anfahren einzelner Messpunkte auf dem zu resezierenden Gewebe zu beschleunigen. Vorteilhaft wird ein sehr schnelles Verfahren geschaffen.

Zweckmäßigerweise wird eine räumliche Position von Messpunkten der Oberfläche anhand einer räumlichen Position vergleichbarer Messpunkte korrigiert, wobei die vergleichbaren Messpunkte aus einem Datensatz einer bildgebenden Aufnahme des zu resezierenden Gewebes stammen.

Zur Qualitätssicherung kann ein von der medizinischen Resektionsvorrichtung ermittelter Oberflächenverlauf mit einem Oberflächenverlauf abgeglichen werden, der durch ein bildgebendes Verfahren wie eine computertomographische Aufnahme ermittelt wurde. Dazu können die beiden Oberflächenverläufe grafisch übereinandergelegt werden und Abweichungen einzelner Oberflächenbereiche voneinander angezeigt werden. Anhand der Unterschiede kann ein Operateur entscheiden, ob eine Korrektur des von der medizinischen Resektionsvorrichtung ermittelten Oberflächenverlaufs erforderlich ist.

Vergleichbare Messpunkte sind solche Messpunkte eines Oberflächenverlauf von zu resezierendem Gewebe, die durch zwei voneinander verschiedene Verfahren ermittelt wurden. Beispielsweise können vergleichbare Messpunkte durch ein bildgebendes Verfahren wie eine computertomographische Aufnahme und durch ein erfindungsgemäßes Verfahren bestimmt werden.

Gerade bei Einsatz von Implantaten zum Ersatz eines menschliches Knies haben die Erfinder die Notwendigkeit erkannt, einen Oberflächenverlauf eines zu resezierenden Knochens durch zwei unterschiedliche Verfahren zu bestimmen, um Fehler wie Messfehler zum minimieren. Durch dieses Vorgehen wird außerdem ein qualitativ besonders hochwertiges Verfahren geschaffen.

In einer Ausgestaltung der Erfindung werden die vergleichbaren Messpunkte aus dem Datensatz der bildgebenden Aufnahme des zu resezierenden Gewebes in ein Bezugssystem der medizinischen Resektionsvorrichtung transformiert.

Vorteilhaft ist ein einfacher Abgleich möglich. Mögliche Abweichungen sind vorteilhaft sofort erkennbar und insbesondere grafisch auf einem Anzeigebildschirm darstellbar.

Zweckmäßigerweise wird durch Differenzbildung von Raumkoordinaten von Messpunkten der Oberfläche und der vergleichbarer Messpunkte des zu resezierenden Gewebes ermittelt, ob eine Korrektur erforderlich ist.

Es werden gleiche Raumpunkte miteinander verglichen. Ein vorteilhaft besonders genaues Verfahren wird geschaffen.

Denkbar ist, dass gleiche Raumpunkte durch Interpolation zwischen benachbarten Raumpunkten bestimmt werden müssen.

In einer Ausgestaltung der Erfindung erfolgen eine Bestimmung einer räumlichen Position und räumlichen Anordnung von zu resezierendem Gewebe und die Resektion durch ein und dieselbe stationäre medizinische Resektionsvorrichtung.

Vorteilhaft wird ein Verfahren geschaffen, bei dem ein und dieselbe stationäre Vorrichtung zur räumlichen Lagebestimmung und zur Resektion verwendbar ist. Dies ist vorteilhaft, da keine Transformation von Raumkoordinaten aus einem Bezugssystem in ein anderes Bezugssystem erforderlich ist, sondern die Raumkoordinaten einer einzigen Vorrichtung ausreichend sind. Es wird ein besonders sicheres Verfahren geschaffen, da Fehler bei Koordinatentransformation aus einem ersten in ein zweites, anderes Bezugssystem ausgeschlossen werden.

In einer weiteren Ausgestaltung der Erfindung erfolgen eine Bestimmung einer räumlichen Position und einer räumlichen Anordnung von zu resezierendem Gewebe und eine Bestimmung und Anzeige von Positionen, an denen ein Resektionshilfsmittel zur teilweisen manuellen Durchführung der Resektion anzubringen ist, durch ein und dieselbe stationäre medizinische Resektionsvorrichtung.

Beispielsweise können nach Bestimmung eines Oberflächenverlaufs Punkte bestimmt werden, an denen das Resektionshilfsmittel, das ein Schnittblock sein kann oder einen solchen umfassen kann, durch Knochenpins mit einem Knochen, der teilweise zu resezieren ist, anzubringen ist.

Denkbar ist, dass das Resektionshilfsmittel anhand des ermittelten Oberflächenverlaufs individuell an einen Patienten angepasst hergestellt wird oder ist. 3D-Druck ist hierfür besonders geeignet.

Es ist außerdem denkbar, dass von der stationären medizinischen Resektionsvorrichtung diejenigen Punkte an zu resezierendem Gewebe angezeigt werden, an denen das Resektionshilfsmittel an dem Gewebe zu fixieren ist. Im Fall einer Sektion von Knochengewebe könnten dies diejenigen Punkte sein, an denen ein Schnittblock durch Knochenpins mit dem Knochen verbunden wird.

Der Erfindung liegt außerdem die Aufgabe zugrunde, eine stationäre medizinische Resektionsvorrichtung auszubilden, durch die ein einstufiges Resektionsverfahren schaffen geschaffen wird, bei dem beide Verfahrensschritte, das heißt eine Bestimmung einer räumlichen Position sowie einer räumlichen Anordnung von zu resezierendem Gewebe und die eigentliche Resektion oder eine Bestimmung und Anzeige von Positionen, an denen ein Resektionshilfsmittel zur teilweise manuellen Durchführung der Resektion anzubringen ist, mittels einer einzigen, das heißt ein und derselben stationären medizinischen Vorrichtung durchführbar sind.

Erfindungsgemäß wird diese Aufgabe durch eine stationäre medizinische Resektionsvorrichtung, insbesondere einen Operationsroboter, gelöst, die zur Durchführung eines vorgenannten erfindungsgemäßen Verfahrens und/oder zur Durchführung eines Verfahrens zur Resektion von Gewebe, insbesondere zur Resektion von Knochengewebe, geeignet ist, bei dem ein Oberflächenverlauf einer Oberfläche von zu resezierendem Gewebe von der medizinischen Resektionsvorrichtung bestimmt wird, indem eine räumliche Position einzelner Messpunkte der Oberfläche des zu resezierenden Gewebes bestimmt wird und aus der räumlichen Position der einzelnen Messpunkte der Oberflächenverlauf ermittelt, wobei der von der medizinischen Resektionsvorrichtung ermittelte Oberflächenverlauf zu deren Ansteuerung zur Resektion des zu resezierenden Gewebes verwendet wird, oder wobei der von der medizinischen Resektionsvorrichtung ermittelte Oberflächenverlauf zur Bestimmung und Anzeige von Positionen eingerichtet ist, an denen ein Resektionshilfsmittel zur teilweisen manuellen Durchführung der Resektion anzubringen ist.

Die Erfinder haben das technische Vorurteil überkommen, wonach zwei getrennte Vorrichtungen und zwei Verfahrensschritte zur Resektion eines Knochens erforderlich sind.

Die Erfinder haben außerdem erkannt, dass eine Referenzierung des Knochens erstens durch die stationäre medizinische Resektionsvorrichtung als solche möglich ist, und zweitens dadurch ein für einen Patienten besonders schonendes Operationsverfahren geschaffen wird, bei dem keine Knochenpins zur Referenzierung in einen Knochen einoperiert werden müssen. Durch die Maschinenführung wird außerdem sichergestellt, dass das Resektionsverfahren standardisierbar ist und nicht vom Geschick eines Operateurs abhängt.

Zweckmäßigerweise umfasst die stationäre medizinische Resektionsvorrichtung eine Steuereinrichtung, die zur Steuerung sowohl einer Bewegung eines Messmittels als auch einer Bewegung eines Resektionsmittels eingerichtet ist, und die zur Bewegung des Messmittels und zur Bewegung des Resektionsmittels ein und dasselbe Bezugssystem heranzieht.

Die Steuereinrichtung kann zum Abarbeiten eines programmierten Messablaufs zur Bestimmung des Oberflächenverlaufs und zum Abarbeiten eines programmierten Resektionsablaufs eingerichtet sein. Dadurch, das ein und dasselbe Bezugssystem, nämlich das Bezugssystem der stationären medizinischen Resektionsvorrichtung, herangezogen wird, ist außerdem keine Koordinatentransformation aus einem Messbezugssystem in ein Resektionsbezugssystem erforderlich.

Dies ist insbesondere dann vorteilhaft, wenn die stationäre medizinische Resektionsvorrichtung ein Operationsroboter ist.

In einer weiteren Ausgestaltung der Erfindung umfasst die stationäre medizinische Resektionsvorrichtung eine Steuereinrichtung, die zur Steuerung sowohl einer Bewegung eines Messmittels als auch einer Bewegung eines Mittels zur Anzeige von Positionen eingerichtet ist, an denen ein Resektionshilfsmittel zur teilweisen manuellen Durchführung der Resektion anzubringen ist.

Das Anzeigemittel kann beispielsweise einen Laser zum Setzen eines Lasermarkierungspunktes oder eine farbstiftähnliche Einrichtung zum Setzen eines Farbpunktes umfassen, an dem beispielsweise ein Knochenpin zur positionsgenauen und positionsstabilen Verbindung eines zu resezierenden Knochens mit dem Resektionshilfsmittel in den Knochen einzubringen ist.

Vorteilhaft kann eine Stelle, an der ein Resektionshilfsmittel anbringbar ist, von der Vorrichtung bestimmt werden, mit der auch der Oberflächenverlauf ermittelt wurde. Eine Koordinatentransformation aus verschiedenen Bezugssystemen ist nicht erforderlich.

In einer Ausgestaltung der Erfindung weist die stationäre medizinische Resektionsvorrichtung ein Messmittel und ein Resektionsmittel auf, wobei das Messmittel einen taktilen oder optischen, vorzugsweise einen taktilen Messkopf umfasst, und das Resektionsmittel ein chirurgisches Operationswerkzeug umfasst.

Durch das Messmittel kann ein Oberflächenverlauf einer Oberfläche von zu resezierendem Gewebe bestimmt werden, während durch das Resektionsmittel eine Resektion des zu resezierenden Gewebes mit derselben Vorrichtung erfolgen kann.

Die Erfinder haben festgestellt, dass eine taktiler Messkopf für medizinische Zwecke besonders gut geeignet ist.

In einer Ausgestaltung der Erfindung sind das Messmittel und das Resektionsmittel an derselben Aufnahmeeinrichtung der stationären medizinischen Resektionsvorrichtung lösbar befestigbar.

Vorteilhaft wird eine stationäre Vorrichtung geschaffen, durch die sowohl ein Oberflächenverlauf als auch eine Resektion unmittelbar nacheinander durchführbar sind. Erforderlich ist lediglich der Austausch des Messmittels durch das Resektionsmittel.

Durch die lösbare Befestigbarkeit ist ein einfacher Austausch möglich.

Die Erfindung wird nachfolgend anhand von Ausführungsbeispielen und der beigefügten, sich auf die Ausführungsbeispiele beziehenden Zeichnungen, näher erläutert. Es zeigen:

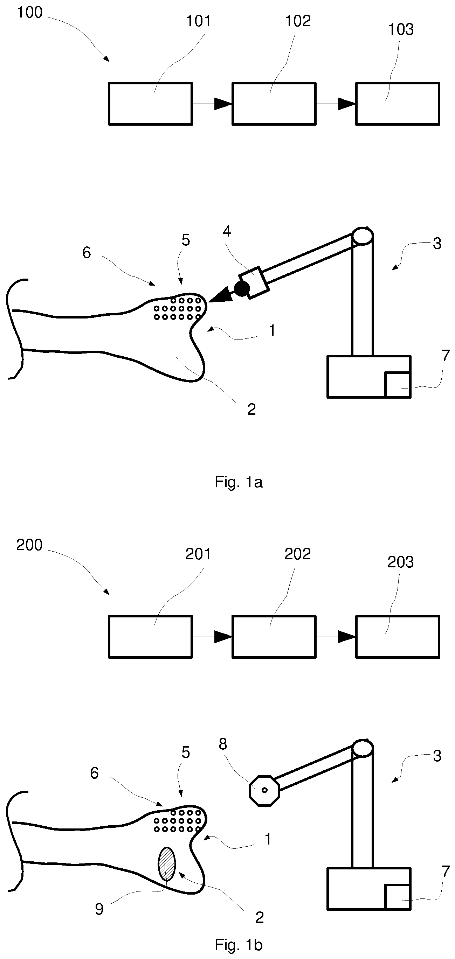

- Fig. 1a-e: Ablauf eines erfindungsgemäßen Verfahrens zur maschinengeführten Bestimmung eines Oberflächenverlaufs einer Oberfläche von zu resezierendem Gewebe sowie zur maschinengeführten Bestimmung von Positionen, an denen ein Resektionshilfsmittel anzubringen ist.

Bei einem in Fig. 1a schematisch gezeigten, vereinfacht dargestellten Verfahren 100 zur Bestimmung eines Oberflächenverlaufs einer Oberfläche 1 von zu resezierendem Knochengewebe 2 werden in einem ersten Verfahrensschritt 101 von einem stationären Operationsroboter 3, der einen taktilen, auswechselbaren Messkopf 4 aufweist, mit dem taktilen Messkopf 4 verschiedene Messpunkte 5, die in diesem Ausführungsbeispiel äquidistant voneinander beanstandet sind, an einer schematisch gezeigten Femurkondyle 6 nacheinander angefahren. Dieses Anfahren erfolgt maschinengeführt. Hierzu umfasst der Operationsroboter eine in Fig. 1a-e nicht gezeigte Steuereinrichtung.

An einem zweiten Verfahrensschritt 102 werden Koordinaten der Messpunkte 5 in einem Bezugssystem des Operationsroboters 3 gespeichert.

In einem dritten Verfahrensschritt 103 wird aus diesen Koordinaten ein Oberflächenverlauf der Femurkondyle 6 durch eine Auswerteinrichtung 7 des Operationsroboters 3 ermittelt. Aus dem Oberflächenverlauf kann eine dreidimensionale, digitale Struktur der Femurkondyle 6 erstellt werden.

Aus Gründen der Übersichtlichkeit sind nicht alle Messpunkte 5 mit einem Bezugszeichen versehen.

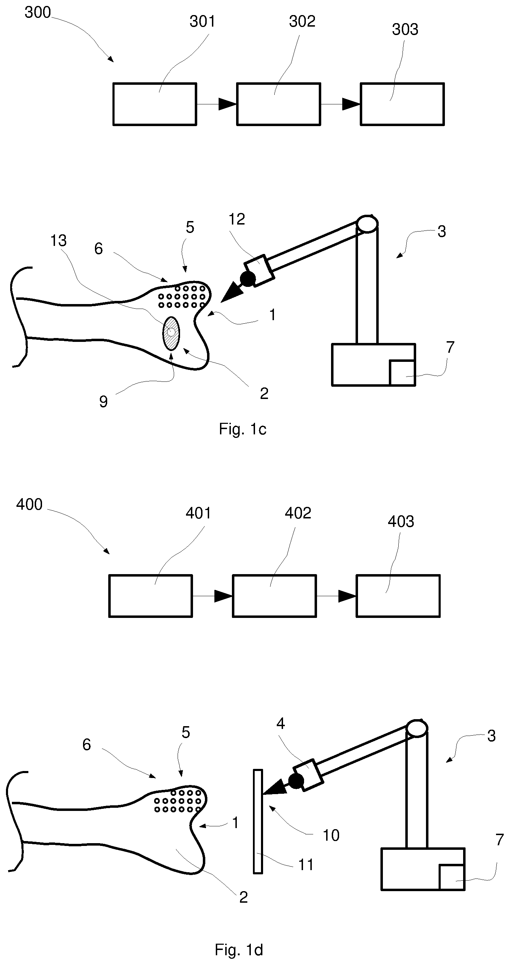

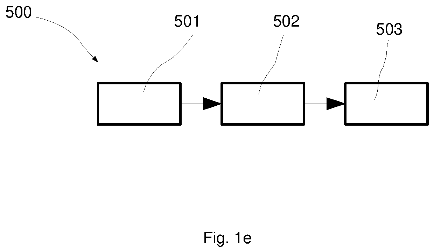

Bei einem in Fig. 1b schematisch gezeigten, vereinfacht dargestellten Verfahren 200 zur Resektion von Knochengewebe 2 wird in einem ersten Verfahrensschritt 201 der taktile Messkopf 4 des Operationsroboters 3 durch ein Operationswerkzeug 8, das eine Säge sein kann, ausgetauscht. Hierzu kann der Operationsroboter 3 eine in Fig. 1 nicht gezeigte Aufnahmeeinrichtung aufweisen.

In einem zweiten Verfahrensschritt 202 wird das Operationswerkzeug 8 an eine von einem Operateur festgelegten Stelle 9 einer freigelegten Femurkondyle 4 bewegt, wobei diese Stelle 9 anhand des Oberflächenverlaufs, der nach dem Verfahren 100 bestimmt wurde, ausgewählt wird.

In einem dritten Verfahrensschritt 203 wird durch das Operationswerkzeug 8 des Operationsroboters 1 an dieser Stelle 7 Knochengewebe 2 der freigelegten Femurkondyle 6 maschinengeführt reseziert.

Dieses Ausführungsbeispiel verdeutlicht, dass ein und dieselbe stationäre Vorrichtung zur Bestimmung des Oberflächenverlaufs und zur Geweberesektion verwendbar sind.

Ferner wird verdeutlicht, dass keine händische Führung des Messkopfes 3 oder des Operationswerkzeugs 8 erforderlich ist. Sämtliche Bewegungen erfolgen maschinengeführt.

Bei einem in Fig. 1c schematisch gezeigten, vereinfacht dargestellten Verfahren 300 zur Bestimmung einer Position 13, an der ein in Fig. 1c nicht gezeigtes Resektionshilfsmittel zur teilweise manuellen Resektion von Knochengewebe 2 anzubringen ist, wird in einem ersten Verfahrensschritt 301 der taktile Messkopf 4 des Operationsroboters 3 durch ein Markierungsmittel 12, das einen Laserpointer umfasst, ausgetauscht. Hierzu kann der Operationsroboter 3 eine in Fig. 1 nicht gezeigte Aufnahmeeinrichtung aufweisen.

In einem zweiten Verfahrensschritt 302 wird das Markierungsmittel 12 in die Nähe einer Stelle 9 einer freigelegten Femurkondyle 4 bewegt, wobei diese Stelle 9 anhand des Oberflächenverlaufs, der nach dem Verfahren 100 bestimmt wurde, ausgewählt wird.

In einem dritten Verfahrensschritt 303 wird durch das Markierungsmittel 12 des Operationsroboters 1 an dieser Stelle 9 die Position 13 markiert, an der ein in Fig. 1c nicht gezeigter Knochenpin zur Befestigung des in Fig. 1c nicht gezeigten Schnittblocks in den Knochen 2 einzubringen ist.

Dieses Ausführungsbeispiel verdeutlicht, dass ein und dieselbe stationäre Vorrichtung zur Bestimmung des Oberflächenverlaufs des zu resezierenden Gewebes und zur Bestimmung und Anzeige von Positionen, an denen ein Resektionshilfsmittel zur teilweisen manuellen Durchführung der Resektion anzubringen ist, verwendbar ist.

Ferner wird verdeutlicht, dass keine händische Führung des Messkopfes 3 oder des Anzeigemittels 12 erforderlich ist. Sämtliche Bewegungen erfolgen maschinengeführt.

Ein in Fig. 1d schematisch gezeigtes Verfahren 400 unterscheidet sich von demjenigen in Fig. 1a gezeigten dadurch, dass drei ein gleichseitiges Dreieck bildende Referenzpunkte 10 in einer Ebene einer ebenen Platte 11, die sich unmittelbar vor einer Femurkondyle 6 befindet, angeordnet sind und von dem Operationsroboter 3 angefahren werden (Verfahrensschritt 401) ist. Durch die drei Referenzpunkte 10, durch die eine Referenzebene für einen Operationsroboter 3 gebildet wird (Verfahrensschritt 402), können von dem taktilen Messkopf 4 Messpunkte 5 aus unmittelbarer Nähe angefahren werden (Verfahrensschritt 403). Es versteht sich, dass die Platte 11 vor Beginn eines Anfahrens mit dem taktilen Messkopf 4 entfernt werden muss.

Ein in Fig. 1e schematisch gezeigtes Verfahren 500 unterscheidet sich von demjenigen in Fig. 1a gezeigten dadurch, dass eine Korrektur einzelner Messpunkte 5 erfolgt.

Dazu werden in einem ersten Verfahrensschritt 501 zu Messpunkten 5 vergleichbare Messpunkte aus einem Datensatz einer computertomographischen Aufnahme in ein Bezugssystem des Operationsroboters 3 transformiert. In einem zweiten Verfahrensschritt 502 wird eine Differenz der Koordinaten von Messpunkten 5 und vergleichbaren Messpunkten der CT-Aufnahme ermittelt. In einem dritten Verfahrensschritt 503 werden bei denjenigen Differenzen, bei denen eine Abweichung von einem festgelegten Grenzwert vorliegt, die Messpunkte 5 dadurch korrigiert, dass diese durch Messpunkte der CT-Aufnahme ersetzt werden.

## Patentansprüche

1. Verfahren zur maschinengeführten Bestimmung eines Oberflächenverlaufs einer Oberfläche (1) von zu resezierendem Gewebe (2), insbesondere eines Oberflächenverlaufs von zu resezierendem Knochengewebe, bei dem eine räumliche Position einzelner Messpunkte (5) der Oberfläche (1) des zu resezierenden Gewebes (2) bestimmt wird, und aus der räumlichen Position der einzelnen Messpunkte der Oberflächenverlauf ermittelt wird,
**dadurch gekennzeichnet,**
**dass** der Oberflächenverlauf von einer stationären medizinischen Resektionsvorrichtung (3) ermittelt wird, die zur Resektion des zu resezierenden Gewebes (2) eingerichtet ist, oder dass der Oberflächenverlauf von einer stationären medizinischen Resektionsvorrichtung (3) ermittelt wird, die zur Bestimmung und Anzeige von Positionen (13) eingerichtet ist, an denen ein Resektionshilfsmittel zur teilweise manuellen Durchführung der Resektion anzubringen ist.

2. Verfahren nach Anspruch 1,
**dadurch gekennzeichnet,**
**dass** aus dem von der stationären medizinischen Resektionsvorrichtung (3) ermittelten Oberflächenverlauf eine dreidimensionale Ansicht des zu resezierenden Gewebes (2) erstellt wird.

3. Verfahren nach Anspruch 1 oder 2,
**dadurch gekennzeichnet,**
**dass** die räumliche Position der einzelnen Messpunkte (5) der Oberfläche taktil bestimmt wird.

4. Verfahren nach einem der Ansprüche 1 bis 3,
**dadurch gekennzeichnet,**
**dass** die räumliche Position der einzelnen Messpunkte (5) und/oder der Oberflächenverlauf in einem Bezugssystem der stationären medizinischen Resektionsvorrichtung bestimmt wird.

5. Verfahren nach einem der Ansprüche 1 bis 4,
**dadurch gekennzeichnet,**
**dass** benachbarte Messpunkte (5) in einem Bezugssystem der medizinischen Resektionsvorrichtung (3) derart ausgewählt werden, dass diese einen Abstand zwischen 1,00 mm und 6,00 mm aufweisen, vorzugsweise zwischen 2,00 mm und 5,00 mm.

6. Verfahren nach einem der Ansprüche 1 bis 5,
**dadurch gekennzeichnet,**
**dass** eine Referenzebene bestimmt wird, die von dem zu resezierenden Gewebe (2) beabstandet ist, und von der aus die räumliche Position der einzelnen Messpunkte (5) der Oberfläche (1) des zu resezierenden Gewebes (2) bestimmt wird.

7. Verfahren nach einem der Ansprüche 1 bis 6,
**dadurch gekennzeichnet,**
**dass** eine räumliche Position von Messpunkten (5) der Oberfläche (1) anhand einer räumlichen Position vergleichbarer Messpunkte korrigiert wird, wobei die vergleichbaren Messpunkte aus einem Datensatz einer bildgebenden Aufnahme des zu resezierenden Gewebes stammen.

8. Verfahren nach Anspruch 7,
**dadurch gekennzeichnet,**
**dass** die vergleichbaren Messpunkte aus dem Datensatz der bildgebenden Aufnahme des zu resezierenden Gewebes in ein Bezugssystem der medizinischen Resektionsvorrichtung (3) transformiert werden.

9. Verfahren nach einem der Ansprüche 1 bis 8,
**dadurch gekennzeichnet,**
**dass** eine Bestimmung einer räumlichen Position und einer räumlichen Anordnung von zu resezierendem Gewebe und die Resektion durch ein und dieselbe stationäre medizinische Resektionsvorrichtung (3) erfolgen.

10. Verfahren nach einem der Ansprüche 1 bis 9,
**dadurch gekennzeichnet,**
**dass** eine Bestimmung einer räumlichen Position und einer räumlichen Anordnung von zu resezierendem Gewebe und eine Bestimmung und Anzeige von Positionen (13), an denen ein Resektionshilfsmittel zur teilweise manuellen Durchführung der Resektion anzubringen ist, durch ein und dieselbe stationäre medizinische Resektionsvorrichtung (3) erfolgen.

11. Stationäre medizinische Resektionsvorrichtung (3), insbesondere Operationsroboter, zur Durchführung eines Verfahrens nach einem der Ansprüche 1 bis 11 und/oder zur Durchführung eines Verfahrens zur Resektion von Gewebe, insbesondere zur Resektion von Knochengewebe, bei dem ein Oberflächenverlauf einer Oberfläche (1) von zu resezierendem Gewebe (2) von der medizinischen Resektionsvorrichtung (3) bestimmt wird, indem eine räumliche Position einzelner Messpunkte (5) der Oberfläche des zu resezierenden Gewebes bestimmt wird und aus der räumlichen Position der einzelnen Messpunkte (5) der Oberflächenverlauf ermittelt, wobei der von der medizinischen Resektionsvorrichtung (3) ermittelte Oberflächenverlauf zu deren Ansteuerung zur Resektion des zu resezierenden Gewebes (2) verwendet wird, oder wobei der von der medizinischen Resektionsvorrichtung (3) ermittelte Oberflächenverlauf zur Bestimmung und Anzeige von Positionen (13) eingerichtet ist, an denen ein Resektionshilfsmittel zur teilweise manuellen Durchführung der Resektion anzubringen ist.

12. Stationäre medizinische Resektionsvorrichtung nach Anspruch 11,
**dadurch gekennzeichnet,**
**dass** die stationäre medizinische Resektionsvorrichtung (3) eine Steuereinrichtung umfasst, die zur Steuerung sowohl einer Bewegung eines Messmittels als auch einer Bewegung eines Resektionsmittels eingerichtet ist, und die zur Bewegung des Messmittels und zur Bewegung des Resektionsmittels ein und dasselbe Bezugssystem heranzieht.

13. Stationäre medizinische Resektionsvorrichtung nach Anspruch 11,
**dadurch gekennzeichnet,**
**dass** die stationäre medizinische Resektionsvorrichtung (3) eine Steuereinrichtung umfasst, die zur Steuerung sowohl einer Bewegung eines Messmittels als auch einer Bewegung eines Mittels (12) zur Anzeige von Positionen (13) eingerichtet ist, an denen ein Resektionshilfsmittel zur teilweise manuellen Durchführung der Resektion anzubringen ist.

14. Stationäre medizinische Resektionsvorrichtung nach Anspruch 11 oder 12,
**dadurch gekennzeichnet,**
**dass** die stationäre medizinische Resektionsvorrichtung (3) ein Messmittel und ein Resektionsmittel aufweist, wobei das Messmittel einen taktilen oder optischen, vorzugsweise einen taktilen Messkopf (4) umfasst, und das Resektionsmittel ein chirurgisches Operationswerkzeug (8) umfasst.

15. Stationäre medizinische Resektionsvorrichtung nach Anspruch 14,
**dadurch gekennzeichnet,**
**dass** das Messmittel (4) und das Resektionsmittel (8) an derselben Aufnahmeeinrichtung der stationären medizinischen Resektionsvorrichtung (3) lösbar befestigbar sind.
